(19) Europäisches Patentamt
European Patent Office
Office européen des brevets

(11) Veröffentlichungsnummer: **0 046 274**
**B1**

(12) # EUROPÄISCHE PATENTSCHRIFT

(45) Veröffentlichungstag der Patentschrift: **08.08.84**

(51) Int. Cl.³: **C 07 D 207/40,** C 07 D 207/416, A 01 N 43/36

(21) Anmeldenummer: **81106296.7**

(22) Anmeldetag: **13.08.81**

(54) **Substituierte Bernsteinsäureimide und ihre Verwendung als fungizide Mittel.**

(30) Priorität: **16.08.80 DE 3030926**

(43) Veröffentlichungstag der Anmeldung: **24.02.82 Patentblatt 82/8**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**US - A - 3 816 451**

**TETRAHEDRON LETTERS, Band 21, Nr. 8, 1980, Pergamon Press, Seiten 705-708 Oxford, New York, Paris, Frankfurt R.A. ABRAMOVITCH et al.: "Pyridinium p-toluenesulfonylmethylide as a formyl anion equivalent"**
**JOURNAL OF PHARMACEUTICAL SCIENCES, vol. 54, (1965) 1257-60**

(73) Patentinhaber: **Consortium für elektrochemische Industrie GmbH, Zielstattstrasse 20, D-8000 München 70 (DE)**

(72) Erfinder: **Häberle, Norman, Dr. Dipl.-Chem., Willibaldstrasse 51 a, D-8000 München 21 (DE)**
Erfinder: **Eberle, Otto, Dr. Dipl.-Ing., Burgmaierstrasse 15, D-8012 Ottobrunn (DE)**

**EP 0 046 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte Bernsteinsäureimide, Verfahren zu deren Herstellung und ihre Anwendung als fungizide Mittel.

Es sind bereits substituierte Bernsteinsäureimide bekanntgeworden, die herbizide oder fungizide Wirksamkeit aufweisen.

Eine zusammenfassende Darstellung zum Thema fungizide Bernsteinsäureimide findet sich in H. Büchel, »Pflanzenschutz und Schädlingsbekämpfung«, 1977, Thieme-Verlag.

Ferner wurden gemäß EP-A-001 395 bereits fungizide Bernsteinsäureimide bekannt, die beispielsweise cyclische Alkenylsubstituenten besitzen.

Weiterhin wurden in der japanischen Anmeldung Kokai 77/61.224 Bernsteinsäureimide mit Alkylthio-alkyl-Seitenketten vorgestellt, die ebenfalls fungitoxische Eigenschaften aufweisen.

Daneben sind gemäß »Journal of Pharmaceutical Sciences«, Vol. 54, No. 9, 1965, 1257-1260 2-Methoxy-N-Phenyl-Bernsteinsäureimid und gemäß »Tetrahedron Letters«, Bd. 21, Nr. 8 (1980) 706 2-Ethoxymethylbernsteinsäure-N-phenylimid beschrieben, ohne daß für die genannten Verbindungen eine fungizide Wirksamkeit bekanntgeworden ist.

Bernsteinsäureimide, die olefinische Doppelbindungen oder chemisch gebundenen Schwefel enthalten, sind jedoch häufig, insbesondere unter Freilandbedingungen, chemisch nicht hinreichend stabil: sie unterliegen Umlagerungsreaktionen bzw. im Fall schwefelhaltiger Bernsteinsäurederivate Oxidations- und Substitutionsreaktionen. Die dabei entstehenden Folgeprodukte sind jedoch nicht mehr ausreichend aktiv.

Aufgabe der Erfindung war es, Bernsteinsäureimide aufzufinden, die bei verbesserter chemischer Stabilität und damit länger anhaltender Wirkung bei Freilandbedingungen ein breites fungizides Wirkungsspektrum aufweisen.

Es wurde nun gefunden, daß eine Auswahl von Bernsteinsäureimiden, die in der 2-Stellung Alkoxy-, Alkoxyalkyl- oder Acyloxy-Substitution aufweisen, die obengenannten Kriterien erfüllen.

Gegenstand der Erfindung sind somit substituierte Bernsteinsäureimide der allgemeinen Formel

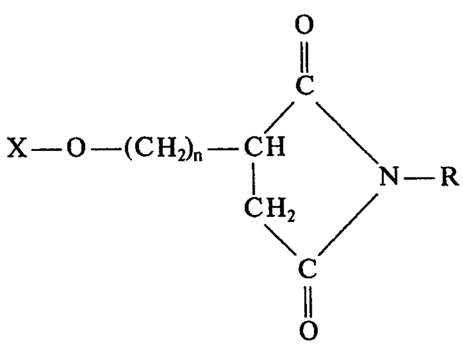

wobei

- n 0 oder 1 ist,
- X einen, ggf. verzweigten Alkylrest mit 1 bis 3 Kohlenstoffatomen, oder, mit der Maßgabe, daß n gleich 0 ist, einen Alkenylrest mit 3 Kohlenstoffatomen oder einen Acylrest mit 2 bis 4 Kohlenstoffatomen, bedeutet und
- R für einen, ggf. verzweigten, Alkylrest mit 1 bis 5 Kohlenstoffatomen, einen Alkenylrest mit 3 bis 5 Kohlenstoffatomen, oder für einen substituierten Phenylrest steht, mit bis zu 2 gleichen oder verschiedenen Substituenten aus der Gruppe der Chlor-, der Brom-, oder der Alkyl- und Alkoxyreste mit 1 bis 3 Kohlenstoffatomen.

Bevorzugt sind solche erfindungsgemäßen Bernsteinsäureimide, in denen R für den 3.5-Dichlorphenylrest steht.

Ein bevorzugtes Verfahren zur Herstellung der erfindungsgemäßen Verbindungen ist dadurch gekennzeichnet, daß Bernsteinsäuren, deren Säurechloride bzw. deren Anhydride, die in der 2-Stellung durch einen

$X-O-(CH_2)_n$-Rest

substituiert sind, wobei

- n 0 oder 1 ist,
- X einen, ggf. verzweigten Alkylrest mit 1 bis 3 Kohlenstoffatomen, oder, mit der Maßgabe, daß n gleich 0 ist, einen Alkenylrest mit 3 Kohlenstoffatomen oder einen Acylrest mit 2 bis 4 Kohlenstoffatomen, bedeutet

mit Aminen der allgemeinen Formel

R—$NH_2$

wobei

R für einen, ggf. verzweigten, Alkylrest mit 1 bis 5 Kohlenstoffatomen, einen Alkenylrest mit 3 bis 5 Kohlenstoffatomen, oder für einen substituierten Phenylrest steht, mit bis zu 2 gleichen oder verschiedenen Substituenten aus der Gruppe der Chlor-, der Brom-, oder der Alkyl- und Alkoxyreste mit 1 bis 3 Kohlenstoffatomen

umgesetzt werden.

Dabei ist es oftmals zweckmäßig, wasser- oder säurebindende Mittel, wie Alkali- oder Erdalkalicarbonate, Alkali- oder Erdalkalihydroxide, oder die analogen Oxide, insbesondere Soda, Pottasche, Magnesia, gebrannten, gelöschten oder kohlensauren Kalk, einzusetzen.

In einer besonders vorteilhaften Verfahrensvariante wird die Umsetzung der Bernsteinsäurederivate mit dem entsprechenden Amin in Gegenwart eines inerten Lösungsmittels, wie beispielsweise Xylol, ausgeführt und das sich bildende Reaktionswasser durch azeotrope Destillation entfernt. Tertiäre Amine, wie Triethylamin oder Pyridin und andere beschleunigen dabei die Wasserabspaltung.

Die zur Herstellung der erfindungsgemäßen Imide benötigten substituierten Bernsteinsäurederivate sind nach grundsätzlich bekannten Verfahren zugänglich. So können Alkoxybernsteinsäuren durch Addition von Alkoholen an Maleinsäure- oder Fumarsäureester synthetisiert werden, wobei zur Vermeidung von Umesterungen und Addition unerwünschter Alkohole an die Doppelbindung der Säuren günstigerweise von solchen Estern ausgegangen wird, deren Alkoholanteil auch an die Doppelbindung addiert werden soll. Aus den Alkoxybernsteinsäureestern sind die freien Säuren durch einfaches Verseifen erhältlich.

Alkoxyalkylbernsteinsäurederivate sind durch Anlagerung von Alkoholen an Itakonsäureester darstellbar, wobei auch hier günstigerweise der gleiche Alkohol zur Veresterung wie auch zur Addition verwendet wird.

Acyloxybernsteinsäurederivate können durch Umsetzung von Äpfelsäure mit einem Überschuß des Carbonsäureanhydrids umgesetzt werden, dessen Acylrest als Substituent gewünscht wird. Ein Überschuß dieses Carbonsäureanhydrids bewirkt dann die Anhydridbildung der so hergestellten Acyloxybernsteinsäuren in einem Reaktionsschritt.

Die Herstellung der einzusetzenden Amine ist ebenfalls bekannt. Die Amine sind in aller Regel im Handel erhältlich.

Beispiele für erfindungsgemäße Bernsteinsäurederivate sind:

2-Methoxy-bernsteinsäure-N-(3-chlorphenyl)-imid
2-Methoxymethyl-bernsteinsäure-N-(3-chlorphenyl)-imid
2-Ethoxy-bernsteinsäure-N-(3-chlorphenyl)-imid
2-Ethoxymethyl-bernsteinsäure-N-(3-chlorphenyl)-imid
2-Propoxy-bernsteinsäure-N-(3-chlorphenyl)-imid
2-iso-Propoxy-bernsteinsäure-N-(3-chlorphenyl)-imid
2-Allyloxy-bernsteinsäure-N-(3-chlorphenyl)-imid
2-Acetoxy-bernsteinsäure-N-(3-chlorphenyl)-imid
2-Propionyloxy-bernsteinsäure-N-(3-chlorphenyl)-imid
2-Methoxy-bernsteinsäure-N-(3.5-dichlorphenyl)-imid
2-Methoxymethyl-bernsteinsäure-N-(3.5-dichlorphenyl)-imid
2-Ethoxy-bernsteinsäure-N-(3.5-dichlorphenyl)-imid
2-Ethoxymethyl-bernsteinsäure-N-(3.5-dichlorphenyl)-imid
2-Propoxy-bernsteinsäure-N-(3.5-dichlorphenyl)-imid
2-iso-Propoxy-bernsteinsäure-N-(3.5-dichlorphenyl)-imid
2-Allyloxy-bernsteinsäure-N-(3.5-dichlorphenyl)-imid
2-Acetoxy-bernsteinsäure-N-(3.5-dichlorphenyl)-imid
2-Propionyloxy-bernsteinsäure-N-(3.5-dichlorphenyl)-imid
2-Methoxy-bernsteinsäure-N-(2.6-dimethylphenyl)-imid
2-Methoxymethyl-bernsteinsäure-N-(2.6-dimethylphenyl)-imid
2-Ethoxy-bernsteinsäure-N-(2.6-dimethylphenyl)-imid
2-Ethoxymethyl-bernsteinsäure-N-(2.6-dimethylphenyl)-imid
2-Allyloxy-bernsteinsäure-N-(2.6-dimethylphenyl)-imid
2-Acetoxy-bernsteinsäure-N-(2.6-dimethylphenyl)-imid
2-Methoxy-bernsteinsäure-N-(3-methoxyphenyl)-imid
2-Methoxymethyl-bernsteinsäure-N-(3-methoxyphenyl)-imid
2-Methoxy-bernsteinsäure-N-butyl-imid
2-Methoxymethyl-bernsteinsäure-N-butyl-imid

2-Methoxymethyl-bernsteinsäure-N-(butyl-2)-imid
2-Ethoxy-bernsteinsäure-N-propyl-imid
2-Methoxymethyl-N-allyl-imid
2-Isobutyroxy-bernsteinsäure-N-allyl-imid
2-Methoxymethyl-bernsteinsäure-N-(3-bromphenyl)-imid
2-Ethoxy-bernsteinsäure-N-(3-bromphenyl)-imid
2-Isopropoxy-bernsteinsäure-N-(3-bromphenyl)-imid

Die erfindungsgemäßen substituierten Bernsteinsäureimide weisen fungitoxische Eigenschaften auf. Sie bewähren sich bei der Bekämpfung pilzlicher Krankheiten. Sie erweisen sich z. B. als hochwirksam gegen Botrytis cinerea (Grauschimmel). Weitere Wirkungsbeispiele sind Pilze wie Alternaria-Arten, Septoria nodorum, Verticillium dahliae, Penicillium glaucum und andere. Weiterhin sind die erfindungsgemäßen Wirkstoffe erfolgreich gegen phytopathogene Pilze, die dem Saatgut anhaften, einsetzbar, wie z. B. Tilletia tritici (Weizensteinbrand), Fusarium nivale, Helminthosporium-Arten.

Die erfindungsgemäßen fungiziden Wirkstoffe eignen sich, ohne daß ihr Anwendungsbereich etwa darauf beschränkt wäre, z. B. zum Einsatz im Weinbau, beim Rapsanbau, in Erdbeerpflanzungen, im Gartenbau, insbesondere in Salatpflanzungen oder bei Zierpflanzen (Alpenveilchen, Geranien usw.). Als weitere erfindungsgemäße Anwendung wurde der Einsatz als Saatgutbeizmittel gefunden.

Die erfindungsgemäßen Wirkstoffe können allein oder im Gemisch mit sonstigen geeigneten Pflanzenschutzmitteln ausgebracht werden. Im allgemeinen werden sie jedoch als Mischungen mit festen oder flüssigen Verdünnungsmitteln oder als Lösungen in festen oder flüssigen Lösungsmitteln verwendet, mit Wirkstoffgehalten von 0,01 bis 95 Gew.-%.

Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 60 Gew.-%, vorzugsweise 15 bis 40 Gew.-% Wirkstoff, 2 bis 25 Gew.-% Dispergierhilfsstoffe und organische Lösungsmittel und/oder Wasser.

Spritzpulver enthalten meistens 10 bis 80 Gew.-%, vorzugsweise 15 bis 70 Gew.-% Wirkstoff, 1 bis 10 Gew.-% Dispergierhilfsstoffe und 10 bis 89 Gew.-% inerte Bestandteile.

Granulate und Stäubemittel enthalten neben inerten Bestandteilen, Bindemitteln und/oder Überzugsstoffen 1 bis 10 Gew.-%, vorzugsweise 5 bis 10 Gew.-% Wirkstoff.

Erfindungsgemäß angewandt werden:

Als Dispergierhilfsstoffe z. B.
  Alkyl- und Arylsulfonate, Methylzellulose, polymere Sulfonsäuren und deren Salze, Polyalkohole, Fettsäureester, Fettalkoholäther, Fettamine;
als organische Lösungsmittel z. B.
  Alkohole, wie Ethanol, Butanole, Dimethylformamid, Dimethylsulfoxid, N-Methylpyrrolidon, Aromaten, wie Toluol und Xylole;
als inerte Bestandteile z. B.
  Kaolin, China-Clay, Talkum, Calciumcarbonat, hochdisperse Kieselsäure, Kieselgele, Kieselgur, Diatomenerde, Bims, Maisschrot, Verdickungsmittel, wie Stärke und Carboxymethylzellulose;
als Bindemittel z. B.
  Magnesiumsulfat, Gips, Gummiarabikum.

Beispielsweise werden die erfindungsgemäßen Fungizide wie folgt formuliert:

1. Emulsionskonzentrat:
   20 Gew.-% Wirkstoff
   10 Gew.-% handelsübliches epoxyliertes Anhydrosorbitmonolaurat (Handelsname »Tween Twenty«)
   70 Gew.-% Dimethylformamid
2. Spritzpulver:
   20 Gew.-% Wirkstoff
   5 Gew.-% Ammoniumligninsulfonat (Handelsname »Totanin«)
   10 Gew.-% Natriumoleylmethyltaurid (Handelsname »Arkopon T Konz«)
   65 Gew.-% Kaolin

Die Aufwandmengen an Wirkstoffen können in großen Bereichen variieren. Bei der Saatgutbehandlung werden im allgemeinen Wirkstoffmengen von 0,05 bis 25 g/kg Saatgut benötigt.

Die Ausbringung der erfindungsgemäßen Wirkstoffe kann in jeder geeigneten Form erfolgen, beispielhaft genannt seien: Gießen, Verspritzen, Versprühen, Verstäuben, Bestreichen, Behandeln des Saatguts (Beizen).

Im folgenden werden die Darstellungsmethoden der erfindungsgemäß substituierten Bernsteinsäuren und ihre fungizide Wirksamkeit anhand von Beispielen erläutert:

## Beispiel 1

### Herstellung von 2-Methoxy-bernsteinsäure-N-(3.5-dichlorphenyl)-imid

a) 360,3 g Fumarsäuredimethylester (2,5 Mol) werden in 80 ml Methanol gelöst. Unter Rühren wird nun langsam eine Lösung von Natriummethylat in Methanol zugetropft. Die Temperatur steigt dabei bis 60° C. Danach wird noch zwei Stunden auf Rückfluß erhitzt, das Methanol abdestilliert und der Rückstand in Wasser gelöst. Nach Ansäuern und Ausäthern werden 422 g Rohprodukt erhalten, von dem ein Festanteil (60 g Ausgangsmaterial) abfiltriert wird. Der Rest wird destilliert und ergibt in 70,4%iger Ausbeute den 2-Methoxy-bernsteinsäure-dimethylester mit einem Siedepunkt von 105 bis 109° C bei 15 Torr. Nach Verseifung mit 2 nNaOH wird die freie 2-Methoxy-bernsteinsäure erhalten. Die freie Säure wird nun durch zweistündiges Erhitzen mit einem Überschuß an Acetanhydrid in 65%iger Ausbeute in das 2-Methoxy-bernsteinsäure-anhydrid ($K_{p\,14}$ 120 bis 123° C) überführt.

b) 130 g (1 Mol) 2-Methoxy-bernsteinsäure-anhydrid und 162 g (1 Mol) 3.5-Dichloranilin werden in 1500 ml Xylol 3 Stunden auf Rückfluß erhitzt. Das entstehende Wasser wird azeotrop mit einem Wasserabscheider abgetrennt. Danach wird das Xylol abdestilliert und das Rohprodukt aus Methanol/Wasser umkristallisiert.
Schmelzpunkt 125 bis 126° C; Ausbeute 75% der Theorie.

## Beispiel 2

### Herstellung von 2-Methoxymethyl-bernsteinsäure-N-(3.5-dichlorphenyl)-imid

a) 200 g Itakonsäuredimethylester werden zusammen mit 5,9 g Natriummethylat in 650 ml Methanol gelöst und 4 Tage stehengelassen. Danach wird mit Essigsäure angesäuert und fraktioniert destilliert. Bei einem Siedebereich von 109 bis 112° C werden 240 g (83,4% der Theorie) an 2-Methoxymethyl-bernsteinsäuredimethylester erhalten. Nach Verseifen des Esters mit 2 nHCl wird die freie Säure in einer Ausbeute von 80,3% der Theorie gewonnen.
Schmelzbereich 94 bis 98° C.

b) 150 g 2-Methoxymethyl-bernsteinsäure, 150 g 3.5-Dichloranilin und 0,5 g Triethylamin werden in 1,5 l Xylol 4 Stunden auf Rückfluß erhitzt und das gebildete Wasser mit einem Wasserabscheider abgetrennt. Danach wird das Lösungsmittel abgezogen. Es fallen 250 g an Zielprodukt in kristalliner Form an.
Schmelzpunkt 95 bis 98° C; Siedepunkt bei 0,1 Torr 155 bis 157° C.

## Beispiel 3

### Herstellung von 2-Acetoxy-bernsteinsäure-N-(3.5-dichlorphenyl)-imid

a) 250 g (1,86 Mol) Äpfelsäure werden in 500 ml Acetanhydrid gelöst und noch 1 ml konzentrierte Schwefelsäure zugesetzt. Das Gemisch wird dann 4 Stunden auf 140° C erhitzt. Schließlich wird im Vakuum eingeengt und der Rest fraktioniert destilliert. Es wird in 60%iger Ausbeute 2-Acetoxy-bernsteinsäureanhydrid erhalten.
Siedepunkt bei 0,01 Torr 108 bis 113° C; Schmelzpunkt 56° C.

b) 158 g (1 Mol) 2-Acetoxy-bernsteinsäureanhydrid und 162 g (1 Mol) 3.5-Dichloranilin werden in 1 l Xylol 3 Stunden auf Rückfluß erhitzt. Das entstehende Wasser wird aceotrop mit einem Wasserabscheider abgetrennt. Danach wird das Xylol abdestilliert und das Rohprodukt aus Essigsäureethylester umkristallisiert. Die Ausbeute an Zielprodukt beträgt 70,3% der Theorie.
Schmelzpunkt 107° C.

## Beispiel 4

### Traubensafttest

20 ml einer Nährlösung aus Traubensaft und destilliertem Wasser im Gewichtsverhältnis 1 : 1 werden in Glas-Petrischalen eingefüllt und mit den in Tabelle 1 angeführten Wirkstoffen in den angegebenen Konzentrationen versetzt. Anschließend werden die Versuchsansätze mit jeweils 50 µl einer Botrytis-Sporensuspension, hergestellt durch Abschwemmen der Botrytis-Sporen von einer Agrarkultur mit destilliertem Wasser, beimpft.

Nach einer Bebrütungsdauer von 10 bzw. 20 Tagen bei 20° C wird das Ausmaß der Pilzentwicklung auf der Nährlösungsoberfläche beurteilt.

Der Wirkungsgrad wird in % nach der folgenden Formel errechnet:

$$100 - \frac{\text{Pilzwachstum, behandelt}}{\text{Pilzwachstum, unbehandelt}} \times 100$$

Tabelle 1

Wirksamkeit gegen Botrytis cinerea im Traubensafttest bei 10 ppm Wirkstoffkonzentration

| Wirkstoff | % Wirksamkeit nach 10 Tagen | % Wirksamkeit nach 20 Tagen |
|---|---|---|
| 2-Methoxy-bernsteinsäure-N-(3.5-dichlorphenyl)-imid | 100 | 100 |
| 2-Ethoxy-bernsteinsäure-N-(3.5-dichlorphenyl)-imid | 100 | 100 |
| 2-Isopropoxy-bernsteinsäure-N-(3.5-dichlorphenyl)-imid | 100 | 100 |
| 2-Allyloxy-bernsteinsäure-N-(3.5-dichlorphenyl)-imid | 80 | 80 |
| 2-Methoxymethyl-bernsteinsäure-N-(3-chlorphenyl)-imid | 90 | 80 |
| 2-Methoxymethyl-bernsteinsäure-N-(3.5-dichlor-phenyl)-imid | 100 | 100 |
| 2-Acetoxy-bernsteinsäure-N-(3.5-dichlorphenyl)-imid | 80 | 70 |

## Vergleichsbeispiel 1

Versuchsanordnung und -durchführung sind die gleichen wie in Beispiel 4 beschrieben.

Tabelle 2

Wirksamkeit gegen Botrytis cinerea im Traubensafttest bei 10 ppm Wirkstoffkonzentration

| Wirkstoff | % Wirksamkeit nach 10 Tagen | % Wirksamkeit nach 20 Tagen |
|---|---|---|
| 2-(Cyclohex-2-en-1-yl)-bernsteinsäure-N-(3.5-dichlorphenyl)-imid | 0 | 0 |
| 2-(Pent-2-en-1-yl-mercapto)-bernsteinsäure-N-(3.5-dichlorphenyl)-imid | 80 | 45 |

Die Vergleichssubstanzen unterscheiden sich von den erfindungsgemäßen Wirkstoffen durch den Substituenten in der 2-Stellung. In einem Fall fehlt die Sauerstoffunktion, im anderen ist sie analog durch chemisch gebundenen Schwefel ersetzt.

Der Vergleich der Ergebnisse gemäß Tabelle 1 und Tabelle 2 zeigt die hohe Wirksamkeit der erfindungsgemäßen Wirkstoffe und ihre anhaltende Wirkung gegenüber den Vergleichsmitteln. Insbesondere wird auch deutlich, daß die Wirkung schwefelhaltiger Präparate nach 20 Tagen stark nachläßt.

## Beispiel 5

### Sporenkeimtest

50 µl einer Lösung oder Suspension des Wirkstoffs mit einem Gehalt von 500 ppm Aktivsubstanz werden zusammen mit 50 µl einer Sporensuspension, hergestellt durch Abschwemmen der Sporen von einer Agrarkultur mit einer Nährlösung, die pro Liter 10 g Zucker, 1 g Glycol, 1 g $KH_2PO_4$ und 0,5 g $MgSO_4$ enthält, in den Hohlschliff von Hohlschliffobjektträgern eingebracht.

Die Objektträger werden bei 20°C 48 Stunden in einer Petrischale, deren Boden mit einem angefeuchteten Filterpapier bedeckt ist, aufbewahrt.

Danach wird das Verhältnis der gekeimten und der nicht gekeimten Sporen gegen eine unbehandelte Kontrollprobe verglichen.

Der Wirkungsgrad wird in % nach der folgenden Formel berechnet:

$$100 - \frac{\text{Anzahl der gekeimten Sporen, behandelt}}{\text{Anzahl der gekeimten Sporen, unbehandelt}} \times 100$$

Die Ergebnisse sind in Tabelle 3 zusammengefaßt:

Tabelle 3

Fungitoxizität von erfindungsgemäßen Wirkstoffen gegen Pilzsporen bei 500 ppm Wirkstoffkonzentration

| Wirkstoff | Alternaria solani | Botrytis cinerea | Fusarium nivale | Penicillium glaucum | Septoria nodorum |
|---|---|---|---|---|---|
| 2-Methoxy-bernsteinsäure-N-(3.5-dichlorphenyl)-imid | 90 | 100 | 90 | 80 | — |
| 2-Ethoxy-bernsteinsäure-N-(3.5-dichlorphenyl)-imid | 70 | 100 | 80 | 80 | — |
| 2-Iso-Propoxy-bernsteinsäure-N-(3.5-dichlorphenyl)-imid | 70 | 100 | 50 | 100 | 50 |
| 2-Allyloxy-bernsteinsäure-N-(3.5-dichlorphenyl)-imid | 80 | 100 | 70 | 80 | 80 |
| 2-Methoxymethyl-bernsteinsäure-N-(3.5-dichlorphenyl)-imid | 100 | 100 | 100 | 100 | 70 |
| 2-Acetoxy-bernsteinsäure-N-(3.5-dichlorphenyl)-imid | 100 | 100 | 100 | 100 | 70 |

## Vergleichsbeispiel 2

Versuchsanordnung und -durchführung sind die gleichen, wie in Beispiel 5 beschrieben. Die Ergebnisse sind in der Tabelle 4 dargestellt.

Tabelle 4

Fungitoxizität von Vergleichswirkstoffen gegen Pilzsporen bei 500 ppm Wirkstoffkonzentration

| Wirkstoff | Alternaria solani | Botrytis cinerea | Fusarium nivale | Penicillium glaucum | Septoria nodorum |
|---|---|---|---|---|---|
| 2-(Cyclohex-2-en-1-yl)-bernsteinsäure-N-(3.5-dichlorphenyl)-imid | 30 | 25 | 0 | 0 | 30 |
| 3-(3.5-Dichlorphenyl)-1-isopropylcarbamoyl-hydantoin Handelsname: Rovral | 0 | 100 | 0 | 60 | 30 |
| 2-(Pent-2-en-1-yl-mercapto)-bernsteinsäure-N-(3.5-dichlorphenyl)-imid | 20 | 20 | 20 | 10 | 10 |

Die Ergebnisse aus Beispiel 5 und Vergleichsbeispiel 2 belegen das verbreiterte Wirkungsspektrum der erfindungsgemäßen Verbindungen gegenüber den Vergleichspräparaten. Diese Eigenschaft ist oft von entscheidender Bedeutung: so treten z. B. neben Botrytis cinerea oft Begleitpilze, wie Alternaria solani und Penicillium glaucum auf. Richtet sich ein Fungizid nur gegen einen bestimmten Pilzstamm, so wird oft das verstärkte Aufwachsen der Begleitpilzart beobachtet. Eine ausreichende Behandlung einer pilzlichen Krankheit ist demnach erst dann gewährleistet, wenn das eingesetzte Fungizid ein derart verbreitertes Wirkungsspektrum besitzt, daß es den größeren Komplex der Pilzkrankheit bekämpft.

Beispiel 6

Saatgutbeizmitteltest

Gesundes Weizensaatgut wird mit Sporen von Tilletia tritici gleichmäßig infiziert. Anschließend wird das infizierte Saatgut mit den als Trockenbeizmittel formulierten erfindungsgemäßen Wirkstoffen sorgfältig gebeizt.

Nach dem Beizen werden die Weizenkörner in feuchte Erdschalen eingelegt. Die Bebrütung erfolgt bei 14 bis 17°C in einem Trockenschrank. Nach 48 Stunden werden die an den Körpern haftenden Sporen durch Eindrücken in Lehm/Erde abgetupft.

Nach weiterer 8tägiger Bebrütung bei 14 bis 17°C im Trockenschrank wird unter dem Binokular das Verhältnis der gekeimten und der nicht gekeimten Sporen im Vergleich zu einer unbehandelten Kontrolle ermittelt.

Die Wirkung der erfindungsgemäßen Wirkstoffe wird in % Keimverhinderung angegeben.

Tabelle 5

Beizmitteltest gegen Weizensteinbrand (Tilletia tritici)

| Wirkstoff | Wirksamkeit in % bei 100 ppm Wirkstoffkonzentration |
|---|---|
| 2-Methoxy-bernsteinsäure-N-(3.5-dichlorphenyl)-imid | 95 |
| 2-Methoxy-bernsteinsäure-N-(4-propylphenyl)-imid | 80 |
| 2-Ethoxy-bernsteinsäure-N-(3.5-dichlorphenyl)-imid | 90 |
| 2-Isopropoxy-bernsteinsäure-N-(3.5-dichlorphenyl)-imid | 100 |
| 2-Allyloxy-bernsteinsäure-N-(3.5-dichlorphenyl)-imid | 100 |
| 2-Methoxymethyl-bernsteinsäure-N-(3-chlorphenyl)-imid | 80 |
| 2-Methoxymethyl-bernsteinsäure-N-(3.5-dichlorphenyl)-imid | 100 |
| 2-Methoxymethyl-bernsteinsäure-N-(butyl-2)-imid | 80 |
| 2-Acetoxy-bernsteinsäure-N-(3.5-dichlorphenyl)-imid | 100 |
| 2-Isobutyroxy-bernsteinsäure-N-allyl-imid | 80 |

## Vergleichsbeispiel 3

Versuchsanordnung und -durchführung sind die gleichen, wie in Beispiel 6 beschrieben. Die Ergebnisse sind in der Tabelle 6 dargestellt.

Tabelle 6

Beizmitteltest gegen Weizensteinbrand (Tilletia tritici)

| Wirkstoff | bei 100 ppm Wirkstoffkonzentration |
|---|---|
| 2-(Cyclohex-2-en-1-yl)-bernsteinsäure-N-(3.5-dichlorphenyl)-imid | 0 |
| 3-(3.5-Dichlorphenyl)-1-isopropylcarbylmoylhydantoin<br>Handelsname: Rovral | 75 |
| 2-(Pent-2-en-1-ylmercapto)-bernsteinsäure-N-(3.5-dichlorphenyl)-imid | 40 |

Der Vergleich der Ergebnisse gemäß Beispiel 6 und Vergleichsbeispiel 3 zeigt erneut die überlegene fungizide Wirksamkeit der erfindungsgemäßen Bernsteinsäurederivate gegenüber den Vergleichspräparaten.

**Patentansprüche für die Vertragsstaaten: BE, CH, DE, FR, GB, IT, LI, NL, SE**

1. Bernsteinsäureimide der allgemeinen Formel

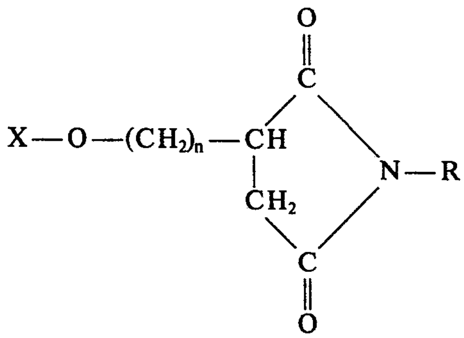

wobei

n 0 oder 1 ist,
X einen, ggf. verzweigten Alkylrest mit 1 bis 3 Kohlenstoffatomen, oder, mit der Maßgabe, daß n gleich 0 ist, einen Alkenylrest mit 3 Kohlenstoffatomen oder einen Acylrest mit 2 bis 4 Kohlenstoffatomen, bedeutet und
R für einen, ggf. verzweigten, Alkylrest mit 1 bis 5 Kohlenstoffatomen, einen Alkenylrest mit 3 bis 5 Kohlenstoffatomen, oder für einen substituierten Phenylrest steht, mit bis zu 2 gleichen oder verschiedenen Substituenten aus der Gruppe der Chlor-, der Brom-, oder der Alkyl- und Alkoxyreste mit 1 bis 3 Kohlenstoffatomen.

2. Bernsteinsäureimide nach Anspruch 1, bei denen R für den 3.5-Dichlorphenylrest steht.
3. Fungizid wirksame Zusammensetzungen, die mindestens ein Bernsteinsäureimid nach den Ansprüchen 1 und 2 enthalten.
4. Verfahren zur Herstellung von Bernsteinsäureimiden gemäß Anspruch 1, dadurch gekennzeichnet, daß Bernsteinsäuren, deren Säurechloride bzw. deren Anhydride, die in der 2-Stellung durch einen

$X-O-(CH_2)_n$-Rest

substituiert sind, wobei n und X die gemäß Anspruch 1 angegebene Bedeutung besitzen mit Aminen der allgemeinen Formel

$R-NH_2$

wobei R die gemäß Anspruch 1 angegebene Bedeutung besitzt umgesetzt werden.
5. Verwendung von Bernsteinsäureimiden der allgemeinen Formel

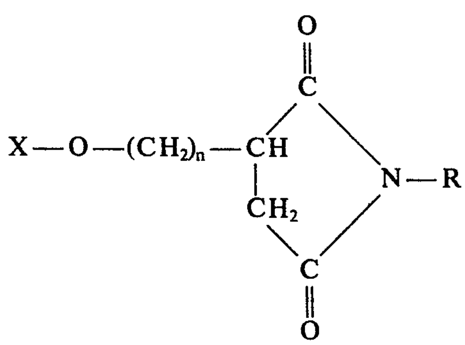

wobei n, X und R die gemäß Anspruch 1 gegebene Bedeutung besitzen, als fungizide Mittel.

**Patentansprüche für den Vertragsstaat: AT**

1. Fungizid wirksame Zusammensetzungen, die mindestens ein Bernsteinsäureimid der allgemeinen Formel

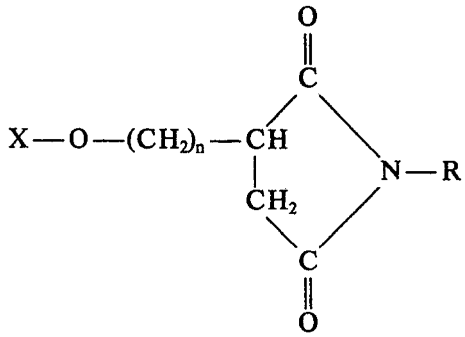

wobei

n 0 oder 1 ist,
X einen, ggf. verzweigten Alkylrest mit 1 bis 3 Kohlenstoffatomen, oder, mit der Maßgabe, daß n gleich 0 ist, einen Alkenylrest mit 3 Kohlenstoffatomen oder einen Acylrest mit 2 bis 4 Kohlenstoffatomen, bedeutet und
R für einen, ggf. verzweigten, Alkylrest mit 1 bis 5 Kohlenstoffatomen, einen Alkenylrest mit 3 bis 5 Kohlenstoffatomen, oder für einen substituierten Phenylrest steht, mit bis zu 2 gleichen oder verschiedenen Substituenten aus der Gruppe der Chlor-, der Brom-, oder der Alkyl- und Alkoxyreste mit 1 bis 3 Kohlenstoffatomen,

enthalten.

2. Fungizid wirksame Zusammensetzungen nach Anspruch 1, die Bernsteinsäureimide enthalten, bei denen R für den 3.5-Dichlorphenylrest steht.

3. Verfahren zur Herstellung von Bernsteinsäureimiden der allgemeinen Formel

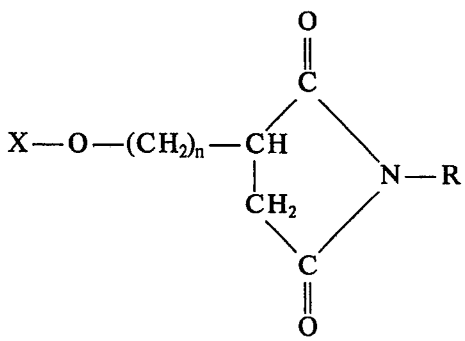

wobei

n 0 oder 1 ist,
X einen, ggf. verzweigten Alkylrest mit 1 bis 3 Kohlenstoffatomen, oder, mit der Maßgabe, daß n gleich 0 ist, einen Alkenylrest mit 3 Kohlenstoffatomen oder einen Acylrest mit 2 bis 4 Kohlenstoffatomen, bedeutet, und
R für einen, ggf. verzweigten Alkylrest mit 1 bis 5 Kohlenstoffatomen, einen Alkenylrest mit 3 bis 5 Kohlenstoffatomen, oder für einen substituierten Phenylrest steht, mit bis zu 2 gleichen oder verschiedenen Substituenten aus der Gruppe der Chlor-, der Brom- oder der Alkyl- und Alkoxyreste mit 1 bis 3 Kohlenstoffatomen,

dadurch gekennzeichnet, daß Bernsteinsäuren, deren Säurechloride bzw. deren Anhydride, die in der 2-Stellung durch einen

$X-O-(CH_2)_n$-Rest

substituiert sind, wobei n und X die oben angegebene Bedeutung besitzen mit Aminen der allgemeinen Formel

$R-NH_2$

wobei R die oben angegebene Bedeutung besitzt, umgesetzt werden.

4. Verwendung von Bernsteinsäureimiden der allgemeinen Formel

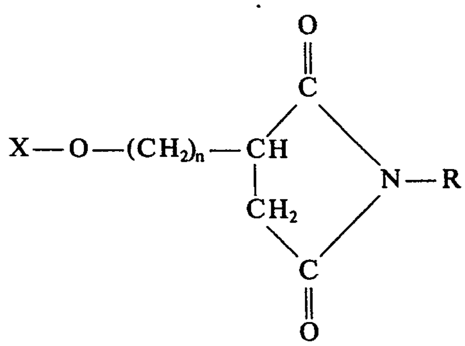

wobei n, X und R die gemäß Anspruch 1 gegebene Bedeutung besitzen, als fungizide Mittel.

**Claims for the Contracting States: BE, CH, DE, FR, GB, IT, LI, NL, SE**

1. Succinic acid imides of the general formula

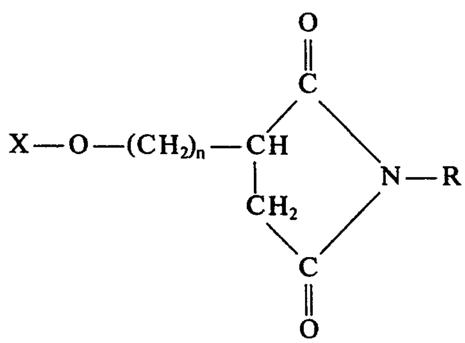

in which

n is 0 or 1,

X represents an optionally branched alkyl radical having from 1 to 3 carbon atoms or, provided that n is 0, an alkenyl radical having 3 carbon atoms or an acyl radical having from 2 to 4 carbon atoms, and

R represents an optionally branched alkyl radical having from 1 to 5 carbon atoms, an alkenyl radical having from 3 to 5 carbon atoms, or a substituted phenyl radical having up to 2 identical or different substituents selected from the group consisting of chlorine atoms, bromine atoms and alkyl and alkoxy radicals having from 1 to 3 carbon atoms.

2. Succinic acid imides according to claim 1, in which R represents the 3,5-dichlorophenyl radical.

3. Fungicidally active compositions containing at least one succinic acid imide according to claim 1 or claim 2.

4. Process for the manufacture of succinic acid imides according to claim 1, characterised in that succinic acids, their acid chlorides or their anhydrides, that are substituted in the 2-position by an

$X-O-(CH_2)_n$-radical,

in which n and X have the meanings given in claim 1, are reacted with amines of the general formula

$R-NH_2$,

in which R has the meaning given in claim 1.

5. Use, as fungicides, of succinic acid imides of the general formula

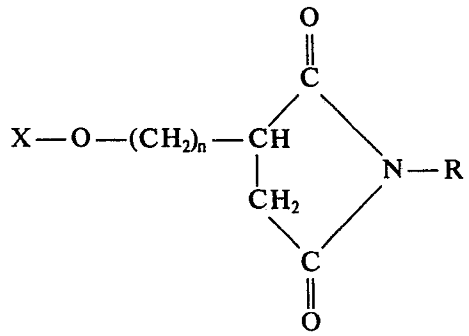

in which n, X and R have the meanings given in claim 1.

**Claims for the Contracting State: AT**

1. Fungicidally active compositions containing at least one succinic acid imide of the general formula

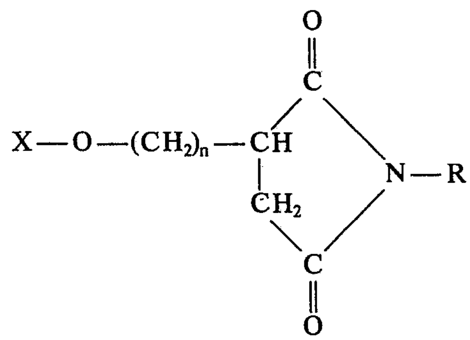

in which

n is 0 or 1,
X represents an optionally branched alkyl radical having from 1 to 3 carbon atoms or, provided that n is 0, an alkenyl radical having 3 carbon atoms or an acyl radical having from 2 to 4 carbon atoms, and
R represents an optionally branched alkyl radical having from 1 to 5 carbon atoms, an alkenyl radical having from 3 to 5 carbon atoms, or a substituted phenyl radical having up to 2 identical or different substituents selected from the group consisting of chlorine atoms, bromine atoms, and alkyl and alkoxy radicals having from 1 to 3 carbon atoms.

2. Fungicidally active compositions according to claim 1 containing succinic acid imides in which R represents the 3,5-dichlorophenyl radical.

3. Process for the manufacture of succinic acid imides of the general formula

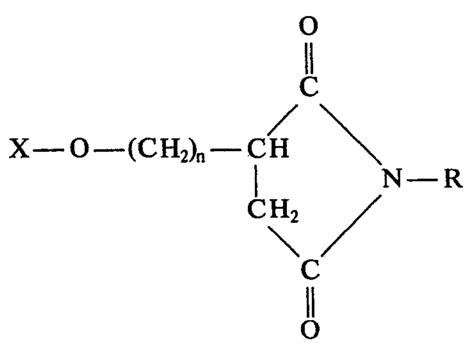

in which

n is 0 or 1,
X represents an optionally branched alkyl radical having from 1 to 3 carbon atoms or, provided that n is 0, an alkenyl radical having 3 carbon atoms or an acyl radical having from 2 to 4 carbon atoms, and

R represents an optionally branched alkyl radical having from 1 to 5 carbon atoms, an alkenyl radical having from 3 to 5 carbon atoms, or a substituted phenyl radical having up to 2 identical or different substituents selected from the group consisting of chlorine atoms, bromine atoms, and alkyl and alkoxy radicals having from 1 to 3 carbon atoms,

characterised in that succinic acids, their acid chlorides or their anhydrides, that are substituted in the 2-position by an

$X-O-(CH_2)_n$-radical,

in which n and X have the meanings given above, are reacted with amines of the general formula

$R-NH_2$,

in which R has the meaning given above.

4. Use, as fungicides, of succinic acid imides of the general formula

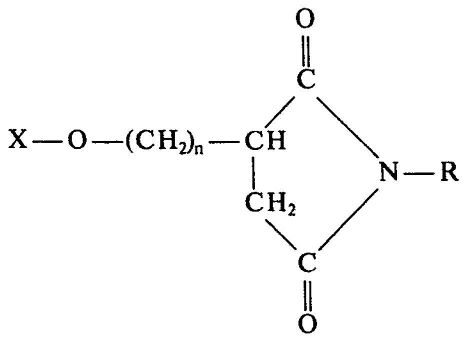

in which n, X and R have the meanings given in claim 1.

**Revendications pour les Etats contractants: BE, CH, DE, FR, GB, IT, LI, NL, SE**

1. Succinimides répondant à la formule générale:

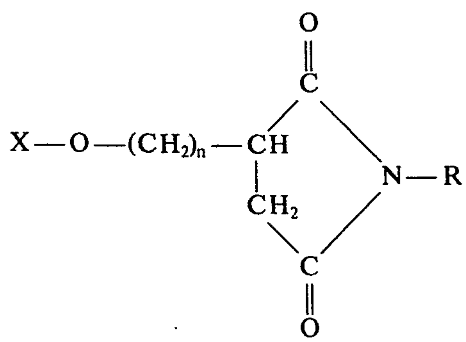

dans laquelle:

n est égal à 0 ou à 1,

X représente un radical alkyle, linéaire ou ramifié, contenant de 1 à 3 atomes de carbone, ou, dans le cas où n est égal à 0, peut représenter un radical alcényle à 3 atomes de carbone ou un radical acyle contenant de 2 à 4 atomes de carbone, et

R représente un radical alkyle, linéaire ou ramifié, contenant de 1 à 5 atomes de carbone, un radical alcényle contenant de 3 à 5 atomes de carbone, ou un radical phényle substitué qui porte au plus deux substituants, identiques ou différents, pris dans l'ensemble constitué par le chlore, le brome et les radicaux alkyles et alcoxy contenant de 1 à 3 atomes de carbone.

2. Succinimides selon la revendication 1 dans lesquels R représente le radical dichloro-3,5 phényle.

3. Compositions fongicides qui contiennent au moins un succinimide selon l'une des revendications 1 et 2.

4. Procédé de préparation de succinimides selon la revendication 1, procédé caractérisé en ce qu'on fait réagir des acides succiniques portant, en position 2, un radical:

$$X-O-(CH_2)_n-$$

dans lequel n et X ont les significations données à la revendication 1, ou des chlorures ou des anhydrides de tels acides, avec des amines répondant à la formule générale:

$$R-NH_2$$

dans laquelle R a la signification donnée à la revendication 1.

5. Application de succinimides répondant à la formule générale:

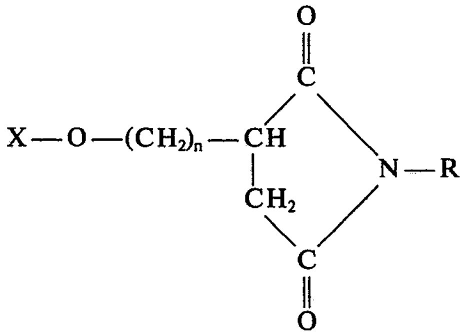

dans laquelle n, X et R ont les significations données à la revendication 1, comme agents fongicides.

**Revendications pour l'Etat contractant: AT**

1. Compositions fongicides qui renferment au moins un succinimide répondant à la formule générale:

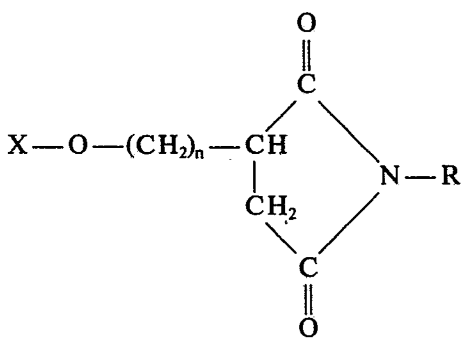

dans laquelle:

- n est égal à 0 ou à 1,
- X représente un radical alkyle, linéaire ou ramifié, contenant de 1 à 3 atomes de carbone, ou, dans le cas où n est égal à 0, peut représenter un radical alcényle à 3 atomes de carbone ou un radical acyle contenant de 2 à 4 atomes de carbone, et
- R représente un radical alkyle, linéaire ou ramifié, contenant de 1 à 5 atomes de carbone, un radical alcényle contenant de 3 à 5 atomes de carbone, ou un radical phényle substitué qui porte au plus deux substituants, identiques ou différents, pris dans l'ensemble constitué par le chlore, le brome et les radicaux alkyles et alcoxy contenant de 1 à 3 atomes de carbone.

2. Compositions fongicides selon la revendication 1, qui contiennent des succinimides dans lesquels R représente le radical dichloro-3,5 phényle.

3. Procédé de préparation de succinimides répondant à la formule générale:

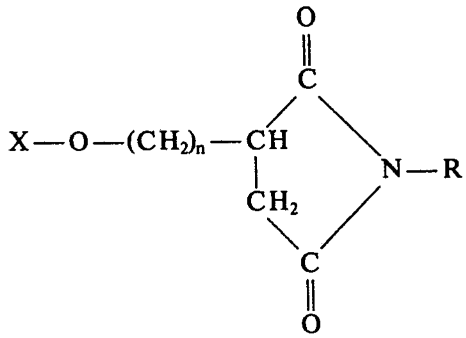

dans laquelle:

n est égal à 0 ou à 1,
X représente un radical alkyle, linéaire ou ramifié, contenant de 1 à 3 atomes de carbone, ou, dans le cas où n est égal à 0, peut représenter un radical alcényle à 3 atomes de carbone ou un radical acyle contenant de 2 à 4 atomes de carbone, et
R représente un radical alkyle, linéaire ou ramifié, contenant de 1 à 5 atomes de carbone, un radical alcényle contenant de 3 à 5 atomes de carbone, ou un radical phényle substitué qui porte au plus deux substituants, identiques ou différents, pris dans l'ensemble constitué par le chlore, le brome et les radicaux alkyles et alcoxy contenant de 1 à 3 atomes de carbone,

procédé caractérisé en ce qu'on fait réagir des acides succiniques portant, en position 2, un radical:

$$X-O-(CH_2)_n-$$

dans lequel n et X ont les significations indiquées plus haut, ou des chlorures ou des anhydrides de tels acides, avec des amines répondant à la formule générale:

$$R-NH_2$$

dans laquelle R a la signification précédemment donnée.

4. Application de succinimides répondant à la formule générale:

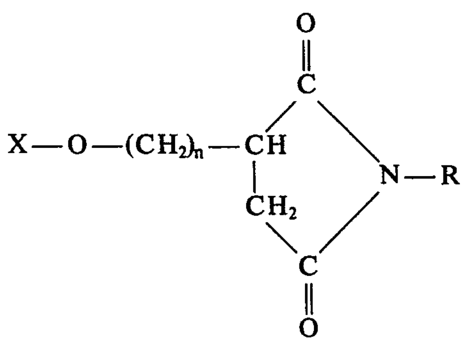

dans laquelle n, X et R ont les significations données à la revendication 1, comme agents fongicides.